# EUROPEAN PATENT APPLICATION

(11) **EP 1 190 702 A1**
(43) Date of publication of application: **27.03.2002**
(21) Application number: 00810874.8
(22) Date of filing: 22.09.2000
(51) Int. Cl.: A61K 7/48, A61K 7/16

(54) **Chitosan emulsion formulation**

(71) Applicant: Ciba Specialty Chemicals Holding Inc., 4057 Basel (CH)
(72) Inventor: Fankhauser, Peter, 4107 Ettingen (CH); Müller, Bernd W., 24220 Flintbek (DE)

(57) **Abstract**

Disclosed is a composition for use as preservative in Home Care or Personal Care products against microbial spoilage comprising
a) 0.1% - 5% of chitosan,
b) 40%-95% of an aqueous phase,
c) 4-40 % of an oily phase,
d) 0.5 - 15% of an emulsifier and
e) optionally other cosmetically acceptable actives, carriers or/and auxiliaries,
and wherein the components have been emulsified to result in a fine emulsion.

The composition is useful for the antimicrobial treatment of skin, mucous membranes or hair, hard surfaces, teeth and gums, as antimicrobial agent and for the preservation of cosmetic and household goods against microbial spoilage.

## Description

Lipid emulsions are known as a drug delivery system with novel properties.

Chitosan is a deacetylated product of chitin obtained from crab or krill and possesses a wide safety margin in comparison to the other positively-charged emulsifying agents which were used to produce positively-charged systems.

In addition to this positive charge, a number of suggestions have been made regarding many interesting properties of chitosan, which may be utilized in many pharmaceutical formulations. One of these properties is the antimicrobial activity of chitosan, which can be utilized as an auxiliary component in a formulation with antimicrobial effects.

Surprisingly it has been found that lipid emulsion formulations improve the antimicrobial efficacy.

Therefore, the present invention relates to the use of chitosan in lipid emulsion formulations to give these formulations antimicrobial properties..

More particularly, the present invention relates to a compositions for use as preservative in Home Care or Personal Care products-against microbial spoilage comprising
a) 0.1% - 5% of chitosan,
b) 40%-95% of an aqueous phase,
c) 4-40 % of an oily phase,
d) 0.5 - 15% of an emulsifier and
e) optionally other cosmetically acceptable actives, carriers or/and auxiliaries,
and wherein the components have been emulsified to result in a fine emulsion.

The chitosan used in the present composition is deacetylated to at least 70%, most preferably to at least 90%.

The chitosan used in the present composition has a low molecular weight, characterized by a viscosity of the aqueous solution at pH 5 of below 30 mPa*s.

Preferably, in the present composition the emulsifier comprises a non-ionic ABA copolymer surfactant (Synperonic F68) or soylecithin (S75).

The chitosan submicron emulsions are prepared by known methods (Jumaa, M.; Müller, B. W. Physicochemical properties of Chitosan-lipid emulsions and their stability during the autoclaving process. *Int. J Phram*. *1999,* 183, 175-184).

Lipid emulsions prepared with chitosan show an enhanced antimicrobial efficacy as demonstrated in the Examples.

Furthermore and particularly, these lipid emulsions display a highly antimycotic efficacy.

The preferred pH-range of the lipid emulsion is from 4.5 to 5.5, and most preferably from 5.0 to 5.5.

The chitosan emulsion formulation according to the invention is also useful for the disinfection and general antimicrobial treatment, such as deodorising, of the skin, mucous membrane and hair, preferably for the disinfection of hands and wounds.

Therefore, formulations according to the invention are suitable as antimicrobially active products in personal care such as skin creams and lotions, refatting shampoos and bath- and shower additives, hair-care products, liquid soaps, deodorants, skin cleaning emulsions and can be used for moist cleaning sheets.

When used as a personal care product, the composition may also comprise further components, e.g. emollients, emulsion stabilisers, skin humectants, skin tanning accelerators, thickeners, such as xanthan, moisture-retention agents, such as glycerol, preservatives, perfumes and colourings.

A further subject of the present invention are also the use of the composition as part of complex products for personal care, household care or oral care comprising at least the chitosan emulsion formulation according to the invention and other suitable components

The complex products according to the present invention comprise 1 -90%, preferably 5 to 90 % b.w. of the chitosan emulsion formulation according to the invention and other suitable components

Cosmetic formulations include a very wide range of cosmetic products. Suitable products are, for example, especially the following:
- skin-care products, for example skin washing and cleansing products in the form of liquid soaps, syndets or washing pastes, skin emulsions, multiple emulsions or skin oils;
- bath products, for example liquid (foam baths, milks, shower products) bath products;
- decorative body-care products, for example face make-ups in the form of day creams, or cream make-ups, eye-care products, for example eye shadow products, mascara, eyeliners, eye creams or eye-fix creams; lip-care products, for example lipstick, lip gloss, lip liner, nail-care products, such nail varnish remover, nail hardeners or cuticle removers;
- feminine hygiene products, such as feminine hygiene washing lotions or sprays;
- foot-care products, for example foot baths, food creams or foot balms, special deodorants and antiperspirants or products for scrubbing off callouses;
- sunscreens, such as sun milks, lotions, creams, sunblockers or tropicals, pre-sun products or after-sun products;
- suntanning products, for example self-tanning creams;
- depigmenting products, for example products for bleaching or lightening skin;
- insect repellents, for example insect oils, lotions, sprays or sticks;
- deodorants, for example deodorant sprays, non-aerosol sprays, deodorant gels, roll-ons;
- antiperspirants, for example creams or roll-ons;
- products for cleansing and treating impure skin, for example liquid syndets, peeling or scrubbing products or peeling masks;
- chemical depilatory products, for example liquid depilatory products, creamy or pasty depilatory products, depilatory gels or aerosol foams;
- shaving products, for example foaming shaving creams, non-foaming shaving creams, shaving foams and gels, preshaving products for dry shaving, aftershaves or aftershave lotions;
- scents, for example perfume creams;
- products for oral and dental hygiene as well as for dentures, for example toothpastes, tooth gels, mouth-wash concentrates, anti-plaque mouth-washes, denture cleaning products or denture adhesion products;
- cosmetic formulations for hair treatment, for example hair washes in the form of shampoos, hair conditioners, hair-care products, for example pretreatment products, hair tonics, hair styling creams and gels, pomades, hair rinses, deep conditioning treatments, intensive hair care treatments, hair setting products, for example hair straightening products, liquid hair fixatives, hair foams, hair sprays,

A shampoo according to the invention has, for example, the following composition
0.1 to 1% by weight of chitosan,
12.0% by weight of sodium laureth-2-sulfate,
4.0% by weight of cocamidopropylbetaine,
3.0% by weight of NaCI and
water to 100%.

A cream deodorant according to the invention has, for example, the following composition
a. oil in water emulsion:
   1.5 %Glyceryl stearate,
   1.0 % Cetyl alcohol,
   1 % Cetearyl alcohol,
   1% PEG-20 stearate,
   1.5 %PEG-7 hydrogenated castor oil,
   3 % C₁₂₋₁₅ alkyl benzoate
   4 %Propylene glycol,
   0.125 % Dimethiconol,
   0.2 % Cyclopentasiloxane,
   0.1-5 % chitosan,
   Water: ad 100%
b. water in silicone emulsion:
   24 % Cyclomethicone,
   3.5 % Dimethicone copolyol,
   3 %Diisopropyl adipate,
   2.75 % Dimethicone/Vinyldimethicone crosspolymer,
   0.4 %Sodium chloride,
   2 %Propylene glycol,
   5 %Glycerin,
   0.1-5 %Chitosan,
   water: add 100%.

The personal care formulations listed above can be in a very wide range of forms of presentation, for example
- in the form of liquid formulations,
- in the form of a gel,
- in the form of an oil, cream, milk or lotion,
- in the form of a spray (spray with propellant or pumping spray) or an aerosol,
- in the form of a foam, or
- in the form of a paste.

The oral hygiene composition may comprise an additional antibacterial enhancing agent, for example an anionic polymeric polycarboxylate, a dehydrated polyphosphate salt, a compound which provides a source of fluoride ions, a polishing material, including siliceous material or sodium bicarbonate, an orally acceptable vehicle, including a water-phase with humectant, thickeners, surface-active agents and a flavoring or sweetening material.

The oral hygiene composition may be in various forms of presentation including the form of a gel, paste, cream or mouthwash.

Furthermore the chitosan emulsion formulation according to the invention according to the invention is useful as household cleaners for the cleaning and disinfection of hard surfaces.

A better understanding of the present invention and of its many advantages will be obtained by referring to the following Examples, given by way of illustration.

### Example 1: Preparation of chitosan submicron emulsions

Chitosan is dispersed in a 5 % aqueous solution of sorbitol to enable isotonicity adjustment and an equal amount of a 2 % (w/v) solution of lactic or acetic acid is added since chitosan is only soluble in an acidic medium. The resulting mixture is stirred vigorously without heating for 60 minutes until chitosan is dissolved. The pH of the resulting solution is 3.6 to 3.8 and this is adjusted to 5.0 using sodium hydroxide (0.1 N) to avoid any flocculation of chitosan. The solution is filtered to separate the non-soluble accompanying fibres. Phospholipids and poloxamer F68 are dissolved in the oil phase by heating. A mixture of castor oil with MCT (1:1) is the oil phase of choice as it was found that this mixture shows low interfacial tension and relatively low viscosity. The emulsions are produced by preparing a premixture of oil phase in aqueous solution using an Ultra-Turrax T25 (Janke & Kunkel, Staufen, Germany) at 8000 rpm for 3 min. The premixture is passed through a high pressure homogenizer (Micron Lab 40, APV Gaulin, Lübeck, Germany) 8 times successively at a pressure of 20 Mpa and a temperature of 40°C. Each emulsion batch is prepared in triplicate. The composition of the tested emulsions is listed in Table 1.

**Table 1:**

| Composition of the tested emulsion formulations | | |
|---|---|---|
| Formulation | Emulsifier (%) | Oil Phase (20%) |
| 1 | S75 (1.5%) | Castor oil- MCT (1:1) |
| 2 | F68 (2%) | Castor oil- MCT (1:1) |
| 3 | F68 (2°/0)+ 0.25 % Chit | Castor oil- MCT (I: I) |
| 4 | F68 (2%)+ 0.5 % Chit | Castor oil- MCT (1:1) |

The mean droplet size of the emulsion is determined by photon correlation spectroscopy (PCS) (Malvern spectrometer RR 102, Malvern, U.K, with Helium- Neon laser λ=632,8 nm, Siemens, Germany) covering the size range 5 nm to approximately 3 µm. Size analysis is studied by adding approximately 1 µl fat emulsion to 1 ml distilled water in order to obtain the optimum scattering intensity. The size of each fat emulsion is analyzed twice and the H₂O:fat samples are analyzed 10 times. The data are expressed in terms of mean diameter and polydispersity factor (deviation of the system from a monodispersed distribution). The mean droplet size of all the emulsion systems prepared is found to be between 150 to 200 nm with the polydispersity factor ranging from 0.15 to 0.2, which indicates a monodispersed distribution.

Larger particles are detected using a laser diffraction analyser LDA (Helos, Sympatec, Clausthal-Zellerfeld, Germany) at a focal length of 20 mm. which corresponds to a measurement range of 0.18 - 35 µm. The emulsions are characterized by the Dₘₐₓ, and the D₅₀ quantiles of the volumetric distribution. D₅₀, mean diameter, is defined as the size in which 50 % of the particles are smaller. Dₘₐₓ is defined as the size in which all particles are smaller. All systems displayed D₅₀ values between 0.57 and 0.63 µm and Dₘₐₓ values are less than 1.8 µm.

The charge on emulsion droplets (ζ potential) is measured using a Zeta Sizer 3 (Malvern Instruments, Malvern, U.K.). The electrolyte solution used for dilution consists of double distilled water with a conductivity of 50 µS/cm adjusted by NaCI (0.5 mmol/L). 500 µL of each emulsion formulation is diluted with 20 mL of the electrolyte solution. Phospholipid, Pluronic and Chitosan-Pluronic emulsions are found to have -55, -10 and +24 mV ζ potential-values, respectively.

### Example 2: Determination of antimicrobial efficacy of the emulsions according to example 1.

Preservation efficacy of emulsion formulations are investigated according to Ph. Eur. 1997, 3ed, chapter 5.1.3.

Ten g emulsion samples are each inoculated with a 0.1 g portion of the suspended microorganisms to obtain an initial challenge number of 10⁵ to 10⁶ cfu per g. The inocula are homogeneously distributed in the samples by gently stirring for 3 minutes and after this the inoculated containers are stored at 25°C. Samples of 1 g are taken for microbial counts from the containers with bacteria after 2, 7 and 28 days, and from the containers with the fungi samples after 14 and 28 days. After dilution of the samples in sodium chloride peptone buffer (pH 7.0), microbial counts are performed using the plate count method on CSA medium for bacteria and on Sabouraud 4 % Dextrose Agar for fungi. The result for each test organism is evaluated as positive when the reduction of the microbial number in the tested emulsion conformed to the requirement of the Pharm. Eur. 99 - preparations for topical applications in case of criterium A.

Subcultures of *Pseudomonas aeruginosa* and *Staphylococcus aureus* are obtained on CSA after incubation for 24 hrs at 35°C. Subcultures of *Candida albicans* and *Aspergillus niger* on Sabouraud 4 % Dextrose Agar at 25° C are obtained after incubation for 48 hrs and 7 days, respectively. The subcultures are washed with aliquots of a sterile solution of 0.9 % sodium chloride in distilled water to prepare inocula with microbial numbers between 10⁷ and 10⁸ cfu per ml. The content of microorganisms in the inocula is determined using absorption measurements at 620 nm. for the bacteria and for *Candida albicans*. The microorganism content of *Aspergillus niger* is determined by microscopic counting using a Thoma chamber. Additionally, the cfu numbers in the inocula are determined by plate counts. These numbers are regarded as the actual concentrations of the inocula.

The antimicrobial efficacy of the lipid emulsion formulations according to the invention are compared with and without chitosan in the composition to prove the effect of chitosan in the formulation.

It is shown in Table 2 that chitosan emulsion formulations reveal a remarkable antimicrobial action while formulations without chitosan display an increased growth of the tested microorganisms.

**Table 2*:**

| Antimicrobial efficacy of different emulsion formulations | | | | |
|---|---|---|---|---|
| Type of emulsion | Antimicrobial efficacy* | | | |
| | A | B | C | D |
| S75(1.5%) | - | - | - | - |
| F68(2%) | - | - | - | - |
| F 68 2 % + 0.25 % Chit. | + | + | + | - |
| F 68 2 °/a + 0.5 % Chit. | + | + | + | + |

| | | | | |
|---|---|---|---|---|
| *) +: conformed to requirements of the preservation efficacy test according to Pharm. Eur. 3, for topical formulations; - : does not conform to the requirements. A Pseudomonas aeruginosa (ATCC 9027) B Staphylococcus aureus (ATCC 6538) C Candida albicans (ATCC 10231) D Aspergillus niger (ATCC 16404) | | | | |

Actually, in chitosan-free formulations an increase in the number of the microorganisms is observed.

Lipid emulsions of example 1 containing chitosan reveal a noticeable antimycotic activity as well, as can be seen from columns C and D in Table 2.

The results in Table 2 show that the lipid emulsion formulation with 0.5 % (w/w) chitosan (with a 92 % degree of deacetylation and low viscosity) leads to a remarkable decrease in the number of the test organisms. The same pattern is observed in 0.25 % chitosan lipid emulsion, except for the unsufficient activity against Aspergillus niger (Table 3).

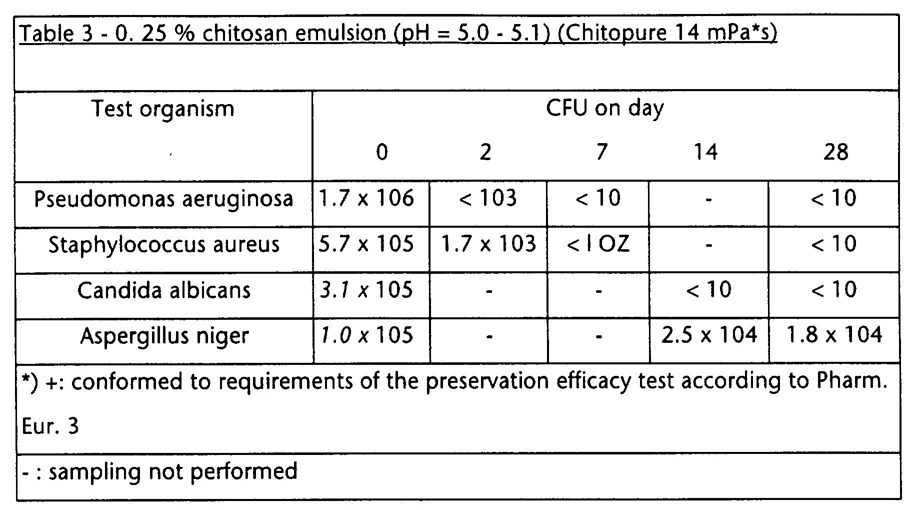

An emulsion containing 0.5 % of chitosan (73 % degree of deacetylation and high viscosity) conforms to the requirements of the preservation efficacy test for topical formulations.

Additionally, the short term effect against the fungi in emulsions containing chitosan with high and low degree of deacetylation and low and high viscosity, respectively is investigated. Both types of chitosan caused reduction in the number of test organisms after 24 hours (Table 4), with the formulation containing chitosan (with 92 % degree of deacetylation and low viscosity) appears to be more active than the other type.

### Example 2: Effect of the type of chitosan on antifungal activity.

Emulsions of example 1 are prepared with two different chitosans with different degree of acetylation and viscosity and tested for antifungal effect as follows:

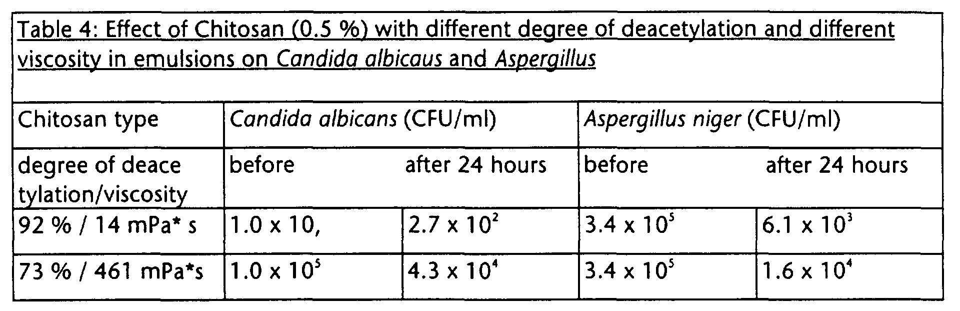

Clearly, the antifungal efficacy is noticeable better for the high desacetylation / low viscosity chitosan.

## Claims

1. Compositions for use as preservative in Home Care or Personal Care products-against microbial spoilage comprising
a) 0.1% - 5% of chitosan,
b) 40%-95% of an aqueous phase,
c) 4-40 % of an oily phase,
d) 0.5 - 15% of an emulsifier and
e) optionally other cosmetically acceptable actives, carriers or/and auxiliaries,
and wherein the components have been emulsified to result in a fine emulsion.

2. Composition according to claim 1 wherein the chitosan is deacetylated to at least 70%

3. Composition according to claim 1 or 2 wherein the chitosan is deacetylated to at least 90%

4. Composition according to any of claims 1 to 3 wherein the chitosan has a low molecular weight, **characterized by** a viscosity of the aqueous solution at pH 5 of below 30 mPa*s

5. Composition according to any of claims 1 to 4 wherein the emulsifier comprises a non-ionic ABA copolymer surfactant.

6. Composition according to any of claims 1 to 4 wherein the emulsifier comprises soy lecithin.

7. Composition according to any of claims 1 to 6 wherein the pH-range of the emulsion is from 4.5 to 5.5.

8. Composition according to claim 7 wherein the pH-range of the emulsion is from 5.0 to 5.5.

9. Use of the composition according to claims 1 - 8 for the antimicrobial treatment of skin, mucous membranes or hair.

10. Use of the composition according to claims 1 - 8 for the antimicrobial treatment of hard surfaces.

11. Use of the composition according to claims 1 - 8 for the antimicrobial treatment of the teeth and gums.

12. Use of the composition according to claims 1 - 8 as antimicrobial agent

13. Use of the composition according to claims 1 - 8 for the preservation of cosmetic and household goods against microbial spoilage.

14. A personal care product comprising at least the emulsion according to claims 1-8.

15. A home care or household product comprising at least the emulsion according to claims 1-8.

16. An oral care product comprising at least the emulsion according to claim 1.

17. The method of stabilizing emulsions against microbial spoilage by use of chitosan.
